# EUROPEAN PATENT SPECIFICATION

(11) **EP 2 822 402 B1**
(45) Date of publication and mention of the grant of the patent: **10.05.2017**
(21) Application number: 13711144.9
(22) Date of filing: 08.03.2013
(51) Int. Cl.: A61P 3/00, A61K 31/198, A61K 31/723, A61K 31/732, A61K 31/736, A23L 2/02, A23L 2/66, A61K 33/06, A23L 33/00, A23L 33/16, A23L 33/175

(54) **GELLED NUTRITIONAL COMPOSITION COMPRISING FREE AMINO ACIDS**
VORGELIERTE NÄHRSTOFFZUSAMMENSETZUNG MIT FREIEN AMINOSÄUREN
COMPOSITION NUTRITIONNELLE GÉLIFIÉE COMPRENANT DES ACIDES AMINÉS LIBRES

(30) Priority: 09.03.2012 WO PCT/NL2012/050147
(43) Date of publication of application: 14.01.2015
(73) Proprietor: N.V. Nutricia, 2712 HM Zoetermeer (NL)
(72) Inventor: LYNCH, Andrew Sean, Liverpool L15 8LX (GB); ANDERS, Simon David, Liverpool L7 9PT (GB)
(74) Representative: V.O.
(86) International application number: PCT/NL2013/050150
(87) International publication number: WO 2013/133711

(56) References cited:
- EP-A1- 0 747 395
- EP-A2- 0 614 616
- WO-A1-2005/096845
- WO-A2-2008/015374
- JP-A- 2009 106 217
- US-A1- 2006 147 495
- US-A1- 2008 021 106
- US-B1- 6 506 422
- ANONYMUS: 'PKU squeezie', [Online] February 2012, Vitaflo Retrieved from the Internet: <URL:http://www.vitaflo.co.uk/blog/2012/02/ > [retrieved on 2015-06-02]
- 'PRODUKTNAME // PZN Zutaten ALLERGENHINWEISE Packungsgröße Dosierung und Anwendung PKU SQUEEZIE // PZN 8468949 Wasser', [Online] 04 April 2016, XP055262602 Retrieved from the Internet: <URL:http://api.gebrauchs.info/974f18e63eb8 df811bcb8ada0d59bb1ai> [retrieved on 2016-04-04]
- MEDPEX APOTHEKE: 'PKU squeezie Gel 30x85 Gramm online bestellen - medpex Versandapotheke', [Online] 04 April 2016, XP055262612 Retrieved from the Internet: <URL:http://www.medpex.de/pku-squeezie-gel- p8468949?q=PKU+sqeezie&pn=1&fs=89.48&fp=1&f op=1> [retrieved on 2016-04-04]

## Description

### Background of the invention

The present invention is in the field of gelled compositions based on amino acids, in particular of such compositions for administration to subjects having an inborn metabolic disease.

The dietary management of many inborn metabolic diseases requires the absence of specific amino acids. *E.g*. phenylketonurea (PKU) patients require protein sources with as low as possible concentrations of phenylalanine. For many years enteral formulas have been developed that are based on free amino acids which are blended into a nutritional complete amino acid composition, but without the specific amino acid such as phenylalanine. Other inborn metabolic diseases may require a different amino acid profile.

These formulas were almost exclusively powder based formulas that the patient could use for mixing with a liquid such as water, lemonade or other low protein liquids. The problem with this is that not all amino acids dissolve easily, and the taste of free amino acid mixtures is often very bad. Since the patients normally require to use these protein sources to supplement their diets with high quality protein without increasing e.g. phenylalanine in the diet during most of their life and in particular during infancy and childhood, these powder products were not convenient enough and also the taste was not good enough for such long term use. Compliance is very important in these patients since not using these products can have severe effects on the growth or if non compliance leads to too high intakes of the amino acid that cannot be metabolized, this may affect neurological development of the patients.

Manufacturers therefore started to develop more convenient ready to feed (rtf) liquid products. These products have an inferior taste and product stability.

US2006/0147495 discloses liquid rtf compositions comprising at least 5wt% amino acids, at least 0.1wt% calcium, and a gelatinizing stabilizer such as guar gum or xanthan. The problem of particle formation was solved by keeping the particles in suspension, preventing the particles to sediment by the increased viscosity of the product. Meaning that the products according to this document still have particles present which in the end will sediment and have a negative impact on taste (sandy particles). This document does not disclose liquid composition with a dry weight of at least 40 g/100 ml. Further, the document teaches away from a composition having a high concentration of free amino acids, because it states that high concentrations of free amino acids will give a very bitter taste. Therefore compositions with high content of free amino acids are said to be unacceptable. PKU Squeezie is an apple and banana flavoured ready-to-feed protein substitute for use in dietary management of phenylketonuria from 6 months to 10 years of age, comprising amino acids, carbohydrates, sugar, vitamins, minerals trace elements and the long chain polyunsaturated fatty acids arachidonic acid and docosahexaenoic acid.

### Summary of the invention

Liquid or gelled (pasteurized or sterilized) products that comprise relatively high amounts of free amino acids suffer from stability problems, in that undesired precipitates are formed. When stored in a container, formed particles settle on the bottom of the container. Also, such particles may adversely affect palatability, and may give rise to a sandy & gritty mouth feel. The inventors have come to the conclusion that in particular amino acids like tyrosine and cysteine, which have a low solubility, form lumps or grains in combination with nutritional micronutrients like calcium and magnesium, resulting in unstable products with poor palatability.

It would be desirable though to provide a well-palatable composition with a satisfactory taste, wherein free amino acids are present as a protein source (*i.e*. a source for *in vivo* protein synthesis). The use of free amino acids as an alternative or in addition to proteins is advantageous, for instance in that it facilitates formulating nutrition for subjects with specific needs, *e.g.* that are in need of a diet wherein one or more amino acids are absent, *e.g.* phenyl alanine for PKU-patients, a diet wherein one or more amino acids are present in a fraction (percentage relative to total amino acid content) that exceeds fractions achievable with proteins, or in case the presence of proteins may be risk in terms of potential allergenic reactions.

In the process of preparing a new amino acid based gelled product the present inventors surprisingly found that if the dry weight of the gelled composition is at least 40% (g/100g) the resulting gelled composition was stable without settling lumps.

Thus, they were able to provide a gelled composition comprising free amino acids in a high concentration that was essentially free of lumps, grains or other particles that may contribute to an unpleasant mouth-feel.

Accordingly, the invention relates to a gelled composition comprising free amino acids, calcium, a digestible carbohydrate and indigestible carbohydrates comprising pectin and at least one indigestible carbohydrate selected from the group of xanthan and guar gum, wherein the free amino acids are present in a concentration between 15 and 30g /100ml, and the dry weight of the total composition is 42-60 wt.%.

A composition according to the invention is in particular suitable for use as a nutrition, more in particular an enteral nutrition.

The nutrition may be for administration to a subject for a non-medical purpose or for a medical purpose.

The present invention is in particular advantageous in that a composition is provided, containing a relatively high concentration of free amino acids, which has a satisfactory shelf-life.

In particular, it has further been surprisingly found that it is possible to provide a composition with a high free amino acid content that not only has a smooth (non-sandy) mouth feel and a satisfactory taste, despite the high free amino acid concentration. In particular, it has been found possible to provide a composition with improved taste, compared to a comparable known free amino acid based liquid or gelled formula. The improved taste of the product is very important for improving the compliance and product loyalty. This is particularly important for patients with -chronic- metabolic diseases that depend for their health on amino acid based formulas.

In an embodiment, the composition is a medical food, *i.e.* a food which is formulated to be consumed or otherwise administered enterally under the supervision of a physician and which is intended for the specific dietary management of a disease or condition for which distinctive nutritional requirements, based on recognizable scientific principles, are established by medical evaluation.

In an embodiment, the invention is directed to a method for treating a subject in need of a composition according to the invention, comprising administering the composition to the subject in an amount providing an effective dosage of the free amino acids contained in the composition.

In a preferred embodiment, the composition of the invention is for use in the treatment of a human suffering from a disorder selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

In a further preferred embodiment the composition is for use in the prophylactic or therapeutic treatment of a neurological disorder in a person suffering from phenylkentonuria.

In a further preferred embodiment, the composition is for use in the treatment of a child suffering from a metabolic disease selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

In a further preferred embodiment, the composition is for use in improving the nutritional status of a subject to which the composition is administered.

In a further preferred embodiment, the composition for use in improving the gut health.

In a further preferred embodiment, the composition is for use in stimulating catch-up growth in a child.

The invention further relates to a method for preparing a composition according to the invention, comprising a multi-stage homogenization step, *i.e,* a homogenization step with at least two stages, wherein the temperature is kept to at least 60 degrees Celsius.

Within the context of the invention, when referred to an administration (to a subject), this is meant to include consumption or other administration to the subject himself, and administration by another, unless specified otherwise.

### Detailed description of the invention

The two main problems with amino acid based gelled products are the bad taste and the bad solubility of several amino acids in combination with calcium and/or phosphate salts. The poor solubility gives rise to the formation of crystals that sink to the bottom of the gelled product. Without being bound by theory, the inventors believe that several amino acids may form insoluble salts when they complex e.g. with calcium or other bivalent cations, normally present in nutritional compositions. In search for a solution of the crystal formation the inventors found that a decrease in water content by increasing the dry weight of the gelled composition significantly decreased crystal formation. In particular it was found if the total amount of amino acids was increased or when the total amount of digestible carbohydrates was increased the crystal size was substantially reduced. Without being bound by theory it is considered that there may be an optimal water concentration wherein crystal formation/growth takes place. To prevent an unacceptable crystal formation, the dry weight is 42-60 wt. %, preferably between 42% and 55%, and most preferably between 42 and 53% based on the total weight of the gelled composition.

There is evidently a relation between the water content and the solubility of salts or amino acids. Adding tyrosine to a highly concentrated solution, i.e. with low water content, is expected to lower the solubility of tyrosine compared with tyrosine dissolved in pure water. Contrary to this expectation, the inventors found that in a solution with decreased water content tyrosine containing crystals were so small that they were not visible anymore.

Within the context of the present invention, "enteral" means any form of administration that involves any part of the gastrointestinal tract, i.e. by mouth (orally), by gastric feeding tube, duodenal feeding tube, or gastrostomy, and rectally, in particular by mouth (orally). Hence, when referring to an enteral composition, this means that the composition is suitable for enteral administration.

With the "dietary management" of an individual is meant the administration of nutritional components to an individual, in such a way that not only the endogenous concentrations of nutritional components are influenced, but that also a health-beneficial effect is obtained.

In accordance with the present invention, liquid products are typically products that are pourable (at 20°C), in particular pourable from an opened container in which they are contained, or that can be withdrawn from a container by sucking (by a person consuming the liquid) through a straw. In particular, a product is liquid or pourable if its viscosity, as measured using the method as described in the definitions section, lies below 2000 mPa.s. Within the context of the invention, the viscosity is the viscosity as measurable using an Anton Paar Physica MCR301 rheometer with a CP50-1/PC cone (diameter 50 mm, 1° difference between middle and outside) at 20°C at 100 s⁻¹. In particular for a liquid product that is intended for administration by drinking, or via a straw or tube, the viscosity preferably is 200 mPa.s or less.

A gelled product is a (previously) liquid product that has been thickened or gelled using specific thickeners such that it cannot be poured anymore without first disturbing the gel network (e.g. a network of polymer chains that form a three dimensional network). Gelled products according to the invention can for instance be administered by spooning.

The term "sandiness" refers to a sensory property of a gelled composition and typically relates to the presence of grains which causes an extraneous feeling remaining on the tongue. This property can be considered as an opposite to 'smoothness' or 'smooth mouth feel' and the absence thereof is an important factor in the acceptance of gelled compositions.

Within the context of the invention, in general the shelf life of a product is the period, starting from its manufacture, during which the product remains suitable for consumption. In particular, during its shelf-life, the product maintains an acceptable microbiological quality, maintains fluidity, and maintains an acceptable mouth-feel. In particular, it remains essentially free of an unacceptable amount of particles that negatively affect the mouth feel.

Within the context of the present invention, the term " sterilization " and the term "pasteurization" is meant to comprise any method to reduce the number of or remove possible pathogens, in particular by using heat or radiation. Preferably, the sterilization treatment includes a heat treatment at a high temperature for a short period, such as a UHT (Ultra High Temperature) treatment.

Within the context of the present invention, a "sterilized or pasteurized composition" is a composition that is obtained or obtainable by subjecting a composition to a sterilization or pasteurization treatment. In general, the quantity of potentially pathogenic micro-organisms of the sterilized or pasteurized composition meets food safety requirements, as applicable *e.g*. in the US or EU. In particular, a sterilized or pasteurized composition in accordance with the invention maintains to meet such requirement, for at least 6 months, preferably at least 12 months, when stored in a sealed packaging at ambient temperature (20 °C).

Within the context of the invention, the term 'free amino acids' is used in general for the proteinogenic amino acids in non-peptidic form (not forming part of a molecule comprising two or more amino acid residues bound via a peptide bond), including salts of free amino acids and esters of free amino acids. In general, the amino acids are thus monomers. Further cystine, a dimer of cysteine is considered a free amino acid, providing cysteine.

A nutritional composition normally comprises the macronutrients fat, carbohydrates and protein equivalents, and micronutrients vitamins, trace elements and minerals. With protein equivalent is meant: proteins, other polypeptides and protein sources for synthesizing proteins, such as free amino acids.

The present invention relates to nutritional compositions comprising free amino acids at least as main protein equivalent. In general, at least 95 wt. % of the protein equivalent is formed by free amino acids. Therefore, in comparison to comparable known liquid or gelled compositions, the water content can be decreased by increasing the content of any of the macronutrients protein equivalent, fat and/or carbohydrates. Salt concentrations cannot be increased, due to taste and osmolarity problems. The concentration of free amino acids can surprisingly be very high.

### Protein source.

The total concentration of the free amino acids should be between 5 and 30 g/100 ml, in particular between 7.5 and 30 g/100ml, preferably between 15 and 25 g/100ml. When increasing the amino acid content to above 30 g/100ml several problems occur. The product will suffer from browning when the concentration is higher than 30 g/100ml. This effect is probably due to reactions between the free amino acids and reducing sugars. The color changes to a very unappealing brown color. In addition, the taste rapidly deteriorates. At a concentration of 25 g/100ml or more the strong bitter amino acid taste already becomes noticeable, but above 30 g/100 ml the taste scores go to unacceptable levels and cannot be masked by the flavors of the other ingredients. The low solubility of tyrosine is thought to be important in the particle formation. The inventors discovered that tyrosine was present in the particles formed in tyrosine containing compositions with less than 40% dry weight.

A suitable concentration for the individual amino acids may be chosen based on known suitable concentrations for a specific independent purpose. For instance, the amino acid composition can be based on generally recommended dosage for an individual, with the proviso that amino acids that cannot be adequately metabolised by the subject for which the composition is intended are typically omitted from the composition.

In a preferred embodiment, the amino acids regarded as essential for a human to which the composition is administered, because he is not capable of synthesising the amino acid himself or not in a sufficient amount, are present in a composition of the invention. In general essential amino acids are phenylalanine, valine, threonine, tryptophan, isoleucine, methionine, leucine, lysine, and histidine. As will be understood by the skilled person, one or more amino acids may be omitted, in view of a subjects medical condition, e.g. in case the subject to be treated with a composition according to the invention has PKU.

Additionally, cysteine, tyrosine, and arginine are required by infants and growing children.

In addition, the amino acids arginine, cysteine, glycine, glutamine, histidine, proline, serine and tyrosine are considered conditionally essential, meaning they are not normally required in the diet, but must be supplied exogenously to specific individuals that do not synthesize it in adequate amounts. An example would be the disease phenylketonuria (PKU). Individuals living with PKU must keep their intake of phenylalanine extremely low to prevent mental retardation and other metabolic complications. However, they cannot synthesize tyrosine from phenylalanine, so tyrosine becomes essential in the diet of PKU patients.

In particular, in accordance with the invention it is considered that the amino acids tyrosine and cystein are conditionally indispensible amino acids for subjects suffering from specific metabolic disorders, depending on specific nutritional treatments, and therefore usually added to nutritional compositions in particular to complete nutritional compositions that should contain all ingredients needed for a healthy diet.

For providing nutritional adequate amounts, the compositions according to the invention preferably have a tyrosine content of at least 1g/100ml, more preferably between 1 and 3 g/100ml and even more preferably between 1.5 and 2.5 g/100ml.

For nutritional purposes, the compositions according to the invention preferably have a cystein content of at least 0.3 g/100ml, more preferably between 0.3 and1.5 g/100ml and even more preferably between 0.4 and 1.0 g/100ml. Usually, the cystein is provided fully or in part in the form of cystin.

As mentioned above, generally at least 95 wt. % of the protein source is present as free amino acid. Preferably 99-100 wt. % of the protein source is present as free amino acid. In particular, a trace of proteins or other polypeptides may be present, in particular originating from an ingredient of the composition that may comprise some protein or other polypeptide, *e.g*. fruit juice.

### Dry weight

Content of dry weight can be calculated by measuring the weight of the gelled product (A) and subsequently drying the product *e.g*. in drying oven or freeze-dry process, and measuring the weight of the dry product (B). The percentage dry weight is then calculated by the ratio B/A and multiplied with 100%. This is a known standard method.

Alternatively, the dry weight can be calculated from the ingredients, taking into account the known moisture content of the ingredients.

As illustrated in the examples, the dry weight should be at least 40 wt.% in order to prevent formation of particles (crystals). Preferably the dry weight is between 40 and 60 wt.%, even more preferably between 41 and 55 wt.% and most preferably between 42 and 50 wt.%.

### Particles/crystals/grains

Particles in the liquid or gelled product result in an unappealing visual deposit and in an unpleasant mouthfeel. With prior art solutions *e.g*. by dispersing the particles with an increase in viscosity of the product, the deposit problem and mouthfeel problem is still noticeable, resulting in an unsatisfactory product. In the examples products are compared comprising thickeners xanthan and gellifier pectin. It appears that only the water content, or dry matter content, is relevant for the decrease in particle formation. Without being bound by theory the inventors believe that the growth of particles, in particular crystals is inhibited at higher dry weight content. Example 3 shows that a clear decrease in particle size is observed when comparing low (35%) and high (42%) dry weight products. It is assumed that particle size above 100 micrometer contribute to a decreased appreciation in mouth feel, and that particles larger than 250 micrometer will be visible in the product and particles above 500 micrometers are really unwanted in a liquid or gelled product.

### Digestible carbohydrates

The concentration of carbohydrates and particularly polymeric carbohydrates can easily be increased without significantly influencing the taste, or affecting the osmolarity negatively. Therefore, a preferred embodiment according to the invention comprises at least 5 g per 100ml, more preferably between 7.5 and 30 g per 100 ml free amino acids, in particular between 7.5 and 25 g per 100 ml free amino acids, more in particular between 10 and 20 g per 100 ml free amino acids free amino acids and at least 10 g per 100 ml digestible carbohydrate.

The digestible carbohydrate content preferably is between 10 and 30 g per 100 ml, more preferably between 15 and 25 g per 100ml.

Glucose or sucrose may suitably be used as digestible carbohydrates, but also fructose, galactose, maltose and combinations thereof can be used as digestible carbohydrates.

A preferred embodiment comprises a digestible polysaccharides, such as maltodextrins, starches, in particular a starch selected from the group of modified starches such as isomaltulose; maize starch, and waxy maize starch. These digestible carbohydrates have the advantage that they do contribute to a lesser content to the osmolarity of the product, than the same weight of a mono- or disaccharide. The osmolarity is relatively high in amino acid based products; therefore a preferred embodiment according to the present invention comprises polysaccharides as digestible carbohydrate. The digestible polysaccharide content as a percentage of the total digestible carbohydrate content can be chosen within a wide range. If present, the content is usually at least 1 wt. %, in particular at least 4 wt. %, at least 10 wt. %, at least 30 wt. %, at least 50 wt. % or at least 70 wt. %.

### Indigestible carbohydrates.

Indigestible carbohydrates have a dual purpose in the present invention. Indigestible carbohydrates are present for their nutritional benefits and because of their effect on the product stability and taste. The inventors believe that the addition of indigestible carbohydrate improves the taste and mouth feel of the product. This is important for improving the compliance of the product.

Preferably the composition comprises pectin as gelatinizer, and Locus bean gum, gellan gum, alginate, carragenaan, xanthan, guar gum or mixtures thereof as stabilizer. Preferably xanthan, guar gum or both are used as stabilizer. In particular pectin has the advantage of increasing the viscosity only after the pasteurization or sterilization heating step and not before cooling down the product. Therefore pipes needed for processing the product are not clogged by the product. This is contrary to many other thickeners that increase viscosity even before heating or during heating, and thereby complicating processing. In addition to that, pectin has excellent gelling characteristics in free amino acid based product and improves the taste. Therefore, in a preferred embodiment the composition of the invention comprises pectin. Preferably the concentration of pectin is between 0.3 and 1.5 g/100ml.

Many children, particularly those with neurological disorders who are often on long term feeds, suffer from constipation. This nutritional purpose is a complex problem and is both distressing to the child and the family involved. For adequate feeding of pediatric patients it is very important to solve the constipation problem in order to improve compliance of the diet. Therefore the present composition according to the invention preferably the composition comprises added dietary fiber.

### Fat

Fat is a concentrated form of energy that contributes little to formula osmolality. As explained above, this is important for amino acid-based compositions. In addition ,it carries fat soluble vitamins and is a source of essential fatty acids. Preferably fat is present between 0 and 50 % of the total energy of the liquid or gelled composition. Preferably the fat is present in a concentration between 0 and 15 g/100 ml, even more preferably between 0.10 and 10 g/100ml and most preferable between 0.30 and 5 g/100ml.

With regard to the type of fat, a wide choice is possible, as long as the fat is of food quality. The fat may either be an animal fat or a vegetable fat or a combination of both. The term fat as used herein includes fatty oils (fats that are liquid at room temperature). Major examples of fats are triglycerides and phospholipids. The fat typically is a source for fatty acids. The fat may include a source of medium chain fatty acids (mainly 8 to 10 carbon atoms long), such as medium chain triglycerides (MCT), a source of long chain fatty acids (mainly at least 18 carbon atoms long), such as poly-unsaturated fatty acids (PUFA's) as omega-3 and omega-6 fatty acids, including EPA, DHA and long chain triglycerides (LCT), and phospholipid-bound fatty acids such as phospholipid-bound EPA or DHA, or any combination of the two types of sources.

The source of fat preferably provides docosahexaenoic acid (DHA). A preferred concentration of DHA is between 0.10 and 0.50 g/100ml.

### Micronutrients

Calcium is an important mineral for infants, children and adults. The daily recommended intake ranges from 200 to 1200 mg/day from infant to adults. It plays an important role in the metabolism and is therefore an essential ingredient in nutritional compositions. Calcium is preferably added to nutritional products as a lactate or phosphate salt, in particular a dicalcium phosphate salt.

The present inventors found that calcium contributed to the particle/crystal formation in the amino acid-based products with a dry weight below 40 %. Surprisingly, calcium can be present in a concentration of 50 mg/100 ml or more, without causing unacceptable particle-formation in a composition according to the invention. Preferably calcium is present in a concentration of at least 50 mg/100ml, even more preferably between 50 and 500 mg/100ml, most preferably between 100 and 500 mg/100ml.

Also magnesium is an important and essential micronutrient. Magnesium is preferably present in a concentration of at least 25 mg/100ml, even more preferably between 25 and 250 mg/100ml and most preferably between 50 and 200 mg/100ml. A preferred magnesium source is magnesium acetate.

### Process for making products according to the present invention.

In an advantageous process for preparing a composition of the invention, the ingredients other than water are first dry blended to provide a blend, This blend is subsequently mixed with water and - if present - other liquid ingredients, after which the blend is pasteurized or sterilized.

The pasteurization or sterilization can be carried out in a manner known pe *se*. In particular good results have been achieved using a heat-treatment, e.g. by pasteurization at about 100 degC for 2-4 secs.

Homogenization is generally carried out after pasteurization or sterilization. Any multi-stage homogenizer can be used according to standard procedures in the food industry. Good results have been achieved with a multi-stage homogenization, wherein a first homogenization stage is carried out, bringing the composition to a first pressure, and in a second homogenization stage the pressure is increased to s second pressure.

The pressure difference between the second and the first state preferably is 25-100 bar, in particular 40-80 bar higher than the first pressure.

The first homogenisation stage is usually performed by bringing the composition to a pressure of 100-250 bar, in particular 150-225 bar. The temperature usually is less than 100 degC, in particular 60-90 degC, more in particular 70-90 degC.

In the second homogenisation stage, the pressure is increased. The temperature in the second stage usually is less than 100 degC, in particular 60-90 degC, more in particular 70-90 degC.

After homogenization, the product is usually packed and cooled to room temperature.

If a temperature-sensitive viscosity modifying agent, such as pectin, is present in the product, it is important that care is taken that the viscosity agent does not cause clogging during the preparation process. The skilled person will generally be able to choose a suitable temperature throughout the process, based on common general knowledge, the information disclosed herein and optionally a limited amount of routine testing. In particular, if pectin is present, it is important to realize that pectin will generally act as a thickening agent or gelling agent only after heating and subsequent cooling of the product. This means that after pasteurization the composition containing pectin should normally be kept above 60 degrees C, preferably between 60 and 90 degrees even more preferably between 70 and 85 degrees Celsius, when homogenizing the product, in order to reduce the risk of clogging.

### Use of the composition according to the present invention

The composition according to the present invention is preferably used for the nutritional management of a subject, in particular a human. A composition according to the invention may in particular be used for administration to a person that has a disorder selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

Preferably the composition according to the invention is for the improvement of nutritional status, the improvement of gut health, or for stimulation of catch-up growth in children suffering from metabolic diseases selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

A composition according to the invention may in particular be used for the treatment of a neurological disorder, such as for the prophylactic or therapeutic treatment of neurological problems that occur in a subject having a metabolic disorder whereby the subject is not capable of adequately metabolizing a specific amino acid (*e.g.* a subject having PKU) or a metabolite of the amino acid. The composition may then in particular be used to avoid the occurrence of an elevated level of an amino acid or metabolite thereof that would cause or contribute to a neurological problem, or to treat a subject having an elevated level of said amino acid or metabolite thereof, in order to decreased the elevated level of the amino acid or metabolite thereof. *E.g*. increased levels of phenylalanine causes brain damage in PKU patients.

Treatment includes in particular nutritional/dietary management. For the present invention this means providing nutrition with a dedicated protein source comprising all necessary amino acids and without the amino acid that cannot be metabolised adequately to a harmless metabolite by a subject for which the composition is intended.

A use of a composition according to the invention generally comprises administration of an effective amount of the composition to a subject in need thereof. The effective amount can generally be based on what is known in the prior art as being effective amounts of amino acids for the intended purpose, the information disclosed herein and optionally a limited amount of routine experimentation.

The composition may in principle be administered in any way. Preferably it is ingested orally or fed into the gastro-intestinal tract by tube feeding.

### Examples

### Process for making products according to the present invention.

First all the dry ingredients were dry blended, an thereafter mixed with water and other liquid ingredients. The resulting blend was pasteurized at 100degC for 2-4secs. Subsequently a 2 stage homogenization was performed in a standard 2-stage homogenizer, at 180-250 bar first stage then increased with 50 bar additional pressure second stage at 70-90 degC. The product was subsequently packed and cooled to room temperature.

### Example 1

Table 1 shows that increasing the dry weight content above 40% the particles/crystals surprisingly disappear using a microscopic evaluation. All ingredients were kept the same, except for the amino acids content. A similar experiment has been done by increasing the corn syrup solids (digestible carbohydrates) while keeping al the other ingredients the same (with 20g amino acids /100ml), resulting in similar effects. The conclusion is that by increasing the dry weight to at least 40% the grains surprisingly disappear from the product thereby improving the stability. The Amino acid mixtures 1-3 together provide a complete amino acid profile as per the group of subjects (patient group) for which the product is intended

**Table 1:**

| **Amino Acid content** | 20g/100ml | | 22.5g/100ml | | 25g/100ml | | 27.5g/100ml | | 30g/100ml | |
|---|---|---|---|---|---|---|---|---|---|---|
| **Water** | 58,78 | 0,00 | 56,19 | 0,00 | 53,60 | 0,00 | 51,01 | 0,00 | 48,42 | 0,00 |
| **Amino acid mix 1** | 14,64 | 14,64 | 16,27 | 16,27 | 18,08 | 18,08 | 19,89 | 19,89 | 21,69 | 21,69 |
| **Juice** | 11,57 | 6,43 | 11,57 | 6,43 | 11,57 | 6,43 | 11,57 | 6,43 | 11,57 | 6,43 |
| **Amino acid mix 2** | 4,82 | 4,82 | 5,42 | 5,42 | 6,03 | 6,03 | 6,63 | 6,63 | 7,23 | 7,23 |
| **Corn syrup solds** | 3,18 | 3,02 | 3,18 | 3,02 | 3,18 | 3,02 | 3,18 | 3,02 | 3,18 | 3,02 |
| **Amino acid mix 3** | 1,43 | 1,43 | 1,61 | 1,61 | 1,79 | 1,79 | 1,97 | 1,97 | 2,15 | 2,15 |
| **Vits Premix** | 1,13 | 1,13 | 1,13 | 1,13 | 1,13 | 1,13 | 1,13 | 1,13 | 1,13 | 1,13 |
| **Calcium salt** | 2,31 | 2,31 | 2,31 | 2,31 | 2,31 | 2,31 | 2,31 | 2,31 | 2,31 | 2,31 |
| **Magnesium salt** | 0,58 | 0,58 | 0,58 | 0,58 | 0,58 | 0,58 | 0,58 | 0,58 | 0,58 | 0,58 |
| **Citric Acid** | 0,56 | 0,56 | 0,56 | 0,56 | 0,56 | 0,56 | 0,56 | 0,56 | 0,56 | 0,56 |
| **Pectin** | 0,69 | 0,61 | 0,69 | 0,61 | 0,69 | 0,61 | 0,69 | 0,61 | 0,69 | 0,61 |
| **Potassium sorbate** | 0,03 | 0,03 | 0,03 | 0,03 | 0,03 | 0,03 | 0,03 | 0,03 | 0,03 | 0,03 |
| **Guar gum** | 0,19 | 0,16 | 0,19 | 0,16 | 0,19 | 0,16 | 0,19 | 0,16 | 0,19 | 0,16 |
| **Xanthan gum** | 0,09 | 0,08 | 0,09 | 0,08 | 0,09 | 0,08 | 0,09 | 0,08 | 0,09 | 0,08 |
| **Sweetener** | 0,02 | 0,02 | 0,02 | 0,02 | 0,02 | 0,02 | 0,02 | 0,02 | 0,02 | 0,02 |
| **Sweetener** | 0,03 | 0,03 | 0,03 | 0,03 | 0,03 | 0,03 | 0,03 | 0,03 | 0,03 | 0,03 |
| **Flavour** | 0,11 | 0,00 | 0,11 | 0,00 | 0,11 | 0,00 | 0,11 | 0,00 | 0,11 | 0,00 |
| **Dry matter g/100g** | | **35,9** | | **38,3** | | **40,9** | | **43,5** | | **46,1** |
| | | | | | | | | | | |
| **Grains** | | ++ | | ++ | | **none** | | **none** | | **none** |

### Example 2a.

**Table 2: product specification in gram.**

| | |
|---|---|
| Water | 51.60 |
| Amino acid 1 | 14.64 |
| Juice | 11.71 |
| Amino acid 2 | 4.88 |
| GS Solids | 11.13 |
| Amino acid 3 | 1.45 |
| Vits Premix | 1.15 |
| TE Premix | 1.15 |
| Citric Acid | 0.56 |
| Pectin | 0.70 |
| Pot sorbate | 0.04 |
| Guar gum | 0.19 |
| Xanthan gum | 0.09 |
| Sunett | 0.00 |
| Sucralose | 0.03 |
| Flavour | 0.11 |
| Magnesium Acetate | 0.58 |

Dry matter content 40.4g/100g

### Example 2b.

**Table 3: Liquid/ non thickened product specification in gram**

| | |
|---|---|
| Water | 51.60 |
| Amino acid 1 | 14.64 |
| Juice | 11.71 |
| Amino acid 2 | 4.88 |
| GS Solids | 11.13 |
| Amino acid 3 | 1.45 |
| Vits Premix | 1.15 |
| TE Premix | 1.15 |
| Citric Acid | 0.56 |
| Pectin | 0 |
| Pot sorbate | 0.04 |
| Guar gum | 0.19 |
| Xanthan gum | 0.09 |
| Sunett | 0.00 |
| Sucralose | 0.03 |
| Flavour | 0.11 |
| Magnesium Acetate | 0.58 |

### Example 3.

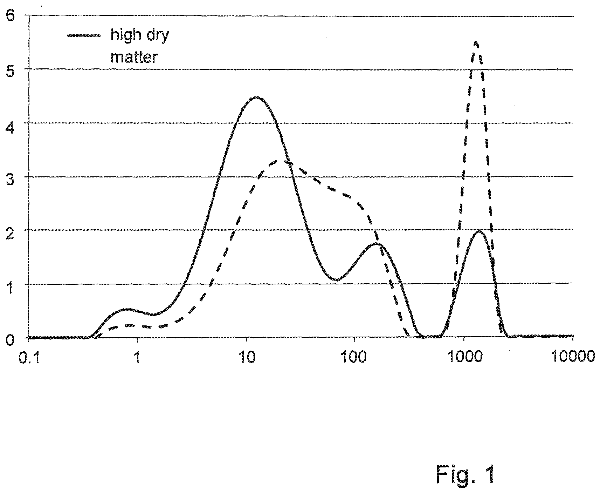

Particle size distribution of a composition with low dry matter (<35%) and high dry matter (>40%) was determined, see Figure 1. On the x-axis the particle size in micrometer and on the y-axis the weight percentage is depicted. Clearly a decreased weight percentage of large particles is visible with high dry matter (solid line) compared to low dry matter (dotted line) content.

The particle size is generated using a Malvern Mastersizer 2000 laser particle size analyser. Procedure was carried out according to the manufacturer's instructions and the following adaptations: The gel in the product was first broken down using a pectinase. Subsequently, an aliquot of this material was dispersed in water and pumped through the measuring cell, with 20,000 laser sweeps being carried out using blue and red lasers. The figure with the size distribution was produces using standard software of the Malvern Mastersizer 2000.

## Claims

1. Gelled pasteurized or sterilized composition comprising free amino acids, calcium , a digestible carbohydrate and indigestible carbohydrates comprising pectin and at least one indigestible carbohydrate selected from the group of xanthan and guar gum, wherein the free amino acids are present in a concentration between 15 and 30 g/100ml, and the dry weight of the total composition is 42 - 60 wt.%.

2. Composition according to claim 1, wherein the digestible carbohydrate content is between 10 and 20 g per 100 ml.

3. Composition according to claim 1 or 2, wherein the free amino acids comprise at least tyrosine in a concentration between 1 - 3 g/100ml.

4. Composition according to any of claim 1-3, wherein the gelled composition comprises fruit juice or fruit puree.

5. Composition according to any of the claims 1-4, wherein the composition further comprises fat, in a concentration between 0.3 and 15 g/100ml.

6. Composition according to any of the previous claims for use as a medicament.

7. Composition for use according to claim 6, wherein the composition is for use in the treatment of a human suffering from a disorder selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

8. Composition for use according to claim 7, for the treatment of a neurological disorder in a person suffering from phenylketonuria.

9. Composition for use according to claim 6, 7 or 8, wherein the composition is for use in the treatment of a child suffering from a metabolic disease selected from the group consisting of phenylketonuria, homocystinuria, maple syrup urine disease, tyrosinaemia, propionic acidaemia, methylmalonic acidaemia, isovaleric acidaemia, urea cycle disorders and glutaric aciduria.

10. Composition for use according to any of claims 6-9, wherein the composition is for improving the nutritional status.

11. Composition for use according to any of claims 6-10, wherein the composition is for improving the gut health.

12. Composition for use according to any of claim 6-11, wherein the composition is for stimulating catch-up growth in a child.

13. Method for preparing a composition according to any of claim 1-5, comprising a multi- stage homogenization step, wherein the temperature is kept to at least 60 degrees Celsius.

14. Method according to claim 13, wherein the temperature is kept from 60 to 90 degrees Celsius.

## Patentansprüche

1. Gelierte, pasteurisierte oder sterilisierte Zusammensetzung, umfassend freie Aminosäuren, Kalzium, ein verdauliches Kohlenhydrat und unverdauliche Kohlenhydrate, umfassend Pektin und mindestens ein unverdauliches Kohlenhydrat, ausgewählt aus der Gruppe von Xanthan und Guargummi, wobei die freien Aminosäuren in einer Konzentration zwischen 15 und 30 g/100 ml vorhanden sind, und das Trockengewicht der Gesamtzusammensetzung 42-60 Gew.-% beträgt.

2. Zusammensetzung nach Anspruch 1, wobei der verdauliche Kohlenhydratgehalt zwischen 10 und 20 g pro 100 ml beträgt.

3. Zusammensetzung nach Anspruch 1 oder 2, wobei die freien Aminosäuren mindestens Tyrosin in einer Konzentration zwischen 1-3 g/100 ml umfassen.

4. Zusammensetzung nach einem der Ansprüche 1-3, wobei die gelierte Zusammensetzung Fruchtsaft oder Fruchtpüree umfasst.

5. Zusammensetzung nach einem der Ansprüche 1-4, wobei die Zusammensetzung ferner Fett in einer Konzentration zwischen 0,3 und 15 g/100 ml umfasst.

6. Zusammensetzung nach einem der vorhergehenden Ansprüche zur Verwendung als ein Arzneimittel.

7. Zusammensetzung zur Verwendung nach Anspruch 6, wobei die Zusammensetzung zur Verwendung bei der Behandlung eines Menschen ist, der an einer Störung leidet, ausgewählt aus der Gruppe bestehend aus Phenylketonurie, Homocystinurie, Ahornsirupharnkrankheit, Tyrosinämie, Propionazidämie, Methylmalonazidämie, Isovalerianazidämie, Harnstoffzyklusstörungen und Glutarazidurie.

8. Zusammensetzung zur Verwendung nach Anspruch 7 für die Behandlung einer neurologischen Störung bei einer Person, die an Phenylketonurie leidet.

9. Zusammensetzung zur Verwendung nach Anspruch 6, 7 oder 8, wobei die Zusammensetzung zur Verwendung bei der Behandlung eines Kindes ist, das an einer Stoffwechselkrankheit leidet, ausgewählt aus der Gruppe bestehend aus Phenylketonurie, Homocystinurie, Ahornsirupharnkrankheit, Tyrosinämie, Propionazidämie, Methylmalonazidämie, Isovalerianazidämie, Harnstoffzyklusstörungen und Glutarazidurie.

10. Zusammensetzung zur Verwendung nach einem der Ansprüche 6-9, wobei die Zusammensetzung zur Verbesserung des Ernährungszustands ist.

11. Zusammensetzung zur Verwendung nach einem der Ansprüche 6-10, wobei die Zusammensetzung zur Verbesserung der Darmgesundheit ist.

12. Zusammensetzung zur Verwendung nach einem der Ansprüche 6-11, wobei die Zusammensetzung zur Stimulierung des Aufholwachstums bei einem Kind ist.

13. Verfahren zur Herstellung einer Zusammensetzung nach einem der Ansprüche 1-5, umfassend einen mehrstufigen Homogenisierungsschritt, wobei die Temperatur auf mindestens 60 Grad Celsius gehalten wird.

14. Verfahren nach Anspruch 13, wobei die Temperatur von 60 bis 90 Grad Celsius gehalten wird.

## Revendications

1. Composition gélifiée pasteurisée ou stérilisée comprenant des acides aminés libres, du calcium, un hydrate de carbone digestible et des hydrates de carbone indigestibles comprenant de la pectine et au moins un hydrate de carbone indigestible choisi dans le groupe de gomme xanthane et guar, dans laquelle les acides aminés libres sont présents dans une concentration de 15 à 30 g/100 ml, la masse sèche de la composition totale est de 42-60 % en masse.

2. Composition selon la revendication 1, dans laquelle la teneur en hydrate de carbone digestible est de 10 à 20 g pour 100 ml.

3. Composition selon la revendication 1 ou 2, dans laquelle les acides aminés libres comprennent au moins de la tyrosine dans une concentration de 1-3 g/100 ml.

4. Composition selon l'une quelconque des revendications 1-3, dans laquelle la composition gélifiée comprend du jus de fruit ou de la purée de fruit.

5. Composition selon l'une quelconque des revendications 1-4, dans laquelle la composition comprend de plus de la graisse, dans une concentration de 0,3 à 15 g/100 ml.

6. Composition selon l'une quelconque des revendications précédentes pour une utilisation comme un médicament.

7. Composition pour une utilisation selon la revendication 6, dans laquelle la composition est destinée à une utilisation dans le traitement d'un être humain souffrant d'un trouble choisi dans le groupe constitué de phénylcétonurie, d'homocystinurie, de maladie des urines à odeur de sirop d'érable, de tyrosinémie, d'acidémie propionique, d'acidémie méthylmalonique, d'acidémie isovalérique, de troubles du cycle de l'urée et d'acidurie glutarique.

8. Composition pour une utilisation selon la revendication 7, pour le traitement d'un trouble neurologique chez une personne souffrant de phénylcétonurie.

9. Composition pour une utilisation selon la revendication 6, 7 ou 8, dans laquelle la composition est destinée à une utilisation dans le traitement d'un enfant souffrant d'une maladie métabolique choisie dans le groupe constitué de phénylcétonurie, d'homocystinurie, de maladie des urines à odeur de sirop d'érable, de tyrosinémie, d'acidémie propionique, d'acidémie méthylmalonique, d'acidémie isovalérique, de troubles du cycle de l'urée et d'acidurie glutarique.

10. Composition pour une utilisation selon l'une quelconque des revendications 6-9, dans laquelle la composition est destinée à améliorer l'état nutritionnel.

11. Composition pour une utilisation selon l'une quelconque des revendications 6-10, dans laquelle la composition est destinée à améliorer la santé de l'intestin.

12. Composition pour une utilisation selon l'une quelconque des revendications 6-11, dans laquelle la composition est destinée à stimuler la croissance de rattrapage chez un enfant.

13. Procédé de préparation d'une composition selon l'une quelconque des revendications 1-5, comprenant une étape d'homogénéisation à étapes multiples, dans lequel la température est maintenue à au moins 60 degrés Celsius.

14. Procédé selon la revendication 13, dans lequel la température est maintenue entre 60 et 90 degrés Celsius.
